# FASCICULE DE BREVET EUROPEEN

(11) **EP 3 390 343 B1**
(45) Date de publication et mention de la délivrance du brevet: **14.10.2020**
(21) Numéro de dépôt: 16815770.9
(22) Date de dépôt: 08.12.2016
(51) Int. Cl.: C07C 69/157, C07C 39/08, A61Q 19/02

(54) **DÉRIVÉS DE RÉSORCINOL POUR LEUR UTILISATION COSMÉTIQUE**
RESORCINDERIVATE ZUR KOSMETISCHEN VERWENDUNG
RESORCINOL DERIVATIVES FOR COSMETIC USE THEREOF

(30) Priorité: 16.12.2015 FR 1562542
(43) Date de publication de la demande: 24.10.2018
(73) Titulaire: L'Oréal, 75008 Paris (FR)
(72) Inventeur: MARAT, Xavier, 93601 Aulnay-sous-Bois (FR); GUEGUINIAT, Amélie, 93601 Aulnay-sous-Bois (FR)
(74) Mandataire: L'Oreal
(86) Numéro de dépôt international: PCT/EP2016/080237
(87) Numéro de publication internationale: WO 2017/102536

(56) Documents cités:
- JP-A- 2007 186 445

## Description

La présente invention concerne l'utilisation cosmétique de composés dérivés de résorcinol pour dépigmenter et/ou blanchir la peau, ainsi que certains composés dérivés de résorcinol nouveaux.

A différentes périodes de leur vie, certaines personnes voient apparaître sur la peau et plus spécialement sur les mains et le visage, des taches plus foncées et/ou plus colorées, conférant à la peau une hétérogénéité. Ces taches sont dues notamment à une concentration importante de mélanine dans les kératinocytes situés à la surface de la peau.

L'utilisation de substances dépigmentantes topiques inoffensives présentant une bonne efficacité est toute particulièrement recherchée en vue de traiter les taches pigmentaires.

Le mécanisme de formation de la pigmentation de la peau, c'est-à-dire de la formation de la mélanine est particulièrement complexe et fait intervenir schématiquement les principales étapes suivantes :
Tyrosine → Dopa → Dopaquinone → Dopachrome → Mélanine

La tyrosinase (monophénol dihydroxyl phénylalanine: oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse, et/ou si elle interfère avec une des étapes de la biosynthèse de la mélanine soit en inhibant une des enzymes impliquées dans la mélanogénèse, soit en s'intercalant comme analogue structural d'un des composés chimiques de la chaîne de synthèse de la mélanine, chaîne qui peut alors être bloquée et ainsi assurer la dépigmentation.

L'arbutine et l'acide kojique sont connus comme agents dépigmentants de la peau.

On a cherché des substances qui présentent une action dépigmentante efficace, notamment supérieure à celle de l'arbutine et de l'acide kojique.

Il subsiste le besoin d'un nouvel agent blanchissant de la peau humaine à action aussi efficace que ceux connus, mais n'ayant pas leurs inconvénients, c'est-à-dire qui soit non irritant, non toxique et/ou non allergisant pour la peau, tout en étant stable dans une composition, ou bien alternativement qui possède une action renforcée de façon à pouvoir être utilisé en quantité plus faible, ce qui diminue considérablement les effets secondaires observés.

A cet égard la Demanderesse a de manière surprenante et inattendue découvert que certains composés de résorcinol présentaient une bonne activité dépigmentante, même à faible concentration, sans faire preuve de cytotoxicité.

A cet égard, la Demanderesse a de manière surprenante et inattendue découvert que certains composés dérivés de résorcinol présentaient une bonne activité dépigmentante, même à faible concentration.

Certains composés dérivés de résorcinol sont déjà connus dans l'art antérieur pour leur activité dépigmentante. A cet égard on peut citer en particulier les documents JP 2007186445 de Kuraray.

L'invention a pour objet des composés de formule (I) tels que définis ci-après pour leur utilisation dermatologique pour dépigmenter la peau.

L'invention a également pour objet de nouveaux composés de formule (II) tels que définis ci-après.

L'invention a aussi pour objet un procédé de traitement cosmétique non thérapeutique de dépigmentation, d'éclaircissement et/ou de blanchiment des matières kératiniques, notamment de la peau, comprenant l'application sur la peau d'au moins un composé de formule (I) telle que définie ci-après.

L'invention a aussi pour objet une composition comprenant, dans un milieu physiologiquement acceptable, au moins un composé de formule (II) telle que définie ci-après.

L'invention a également pour objet un procédé cosmétique non thérapeutique de dépigmentation, d'éclaircissement et/ou de blanchiment des matières kératiniques, notamment de la peau, comprenant l'application de la composition décrite précédemment.

Plus préférablement, il s'agit du procédé pour dépigmenter, éclaircir et/ou blanchir la peau.

L'invention concerne également l'utilisation cosmétique non thérapeutique d'au moins un composé de formule (I) telle que définie ci-après comme agent blanchissant, éclaircissant et/ou dépigmentant des matières kératiniques, notamment de la peau.

Les composés conformes à l'invention, à savoir en particulier de formule (I) ou de formule (II) telles que définies ci-après, permettent de dépigmenter et/ou d'éclaircir efficacement, voire de blanchir, la peau d'êtres humains. Ils sont notamment destinés à être appliqués sur la peau d'individus présentant des taches de pigmentation brunâtres, des taches de sénescence, ou sur la peau d'individus désirant combattre l'apparition d'une couleur brunâtre provenant de la mélanogénèse.

Ils peuvent également permettre de dépigmenter et/ou d'éclaircir les poils, les cils, les cheveux, ainsi que les lèvres et/ou les ongles.

L'invention a donc pour objet des composés de formule (I) comme suit, pour leur utilisation pour dépigmenter, éclaircir et/ou blanchir la peau : 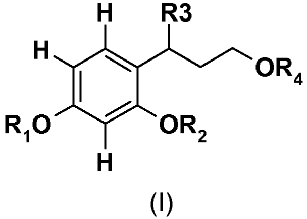 dans laquelle:
R₁, R₂ : identiques ou différents, désignent
   a) hydrogène,
   b) un radical COR₅ dans lequel R₅ désigne un radical alkyle linéaire en C₁-C₁₀ ou ramifié en C₃-C₁₀, de préférence un radical alkyle linéaire en C₁-C₆ ou ramifié en C3-C6, plus préférentiellement un radical alkyle linéaire en C₁-C₄
R₃ désigne un radical alkyl linéaire en C₁-C₆ ou ramifié en C₃-C₆, de préférence un radical alkyle linéaire en C₁-C₄ ou ramifié en C₃-C₄,
R₄ désigne
   a) H,
   b) un radical alkyl linéaire en C₁-C₆ ou ramifié en C₃-C₆,
   c) un radical COR₅,
ainsi que leurs sels, leurs solvates et leurs isomères optiques, leurs racémiques, seuls ou en mélange.

De préférence R₁ et R₂ sont identiques.

De préférence R₄ désigne H ou un radical alkyl linéaire en C₁-C₄, un radical alkyl ramifié en C₃-C₄ ou un radical COR₅, plus préférentiellement H ou un radical COR₅.

Préférentiellement, les composés de formule (I) sont choisis parmi ceux pour lesquels :
R₁, R₂, identiques ou différents, désignent H ou un radical COCH₃, et de préférence R₁ et R₂ sont identiques.
R₃ désigne méthyle, éthyle ou isopropyle, et
R₄ désigne H ou un radical COCH₃.
ainsi que leurs sels, leurs solvates et leurs isomères optiques, leurs racémiques, seuls ou en mélange.

Plus préférentiellement, les composés de formule (I) sont tels que :
R₁, R₂, identiques ou différents, désignent H ou un radical COCH₃, et de préférence R₁ et R₂ sont identiques.
R₃ désigne méthyle, et
R₄ désigne H ou un radical COCH₃,
ainsi que leurs sels, leurs solvates et leurs isomères optiques, leurs racémiques.

Toujours dans le cadre de la présente invention, les sels des composés de formule (I) telle que définie ci-après comprennent les sels non toxiques conventionnels desdits composés tels que ceux formés à partir d'acide ou de base.

Comme sels du composé de formule (I) on peut citer :
les sels obtenus par addition du composé de formule (I) (lorsqu'il comprend un groupe acide) avec une base minérale, telle que la soude, la potasse, l'hydroxyde de calcium, l'hydroxyde d'ammonium, l'hydroxyde de magnésium, l'hydroxyde de lithium, et les carbonates ou hydrogénocarbonates de sodium, de potassium ou de calcium par exemple ;
ou avec une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, l'éthanolamine, la triéthanolamine, la diméthylamino-2-propanol, le 2-amino-2-(hydroxyméthyl)-1,3-propanediol, la 3-(diméthylamino) propylamine.

On peut encore citer les sels d'acides aminés tels que par exemple, la lysine, l'arginine, la guanidine, l'acide glutamique, l'acide aspartique. Avantageusement, les sels des composés de formule (I) (lorsqu'il comprend un groupe acide) peuvent être choisis parmi les sels alcalins ou alcalinoterreux tels que sodium, potassium, calcium, magnésium ; les sels d'ammonium.

Les solvates acceptables des composés décrits dans la présente invention comprennent des solvates conventionnels tels que ceux formés lors de la préparation desdits composés du fait de la présence de solvants. A titre d'exemple, on peut citer les solvates dus à la présence d'eau ou d'alcools linéaires ou ramifiés comme l'éthanol ou l'isopropanol.

Les isomères optiques sont notamment les énantiomères et les diastéréoismères.

Toujours dans le cadre de la présente invention :
Sans indication contraire, un « groupe (Cₓ-C_{y})alkyle » désigne un groupe alkyle linéaire et saturé comprenant de x à y atomes de carbone.

De façon préférentielle, les groupes alkyles linéaires saturés ou ramifiés peuvent être choisis parmi : méthyle, éthyle, propyle, isopropyle, butyle, isobutyle, tertio-butyle, pentyle, hexyle.

Les composés de formule (I) particulièrement préférés sont choisis parmi :

| Structure | Composé n° | | Nom chimique |
|---|---|---|---|
| 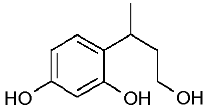 | 1 | | 4-(3-Hydroxy-1-méthyl-propyl)-benzène-1,3-diol |
| 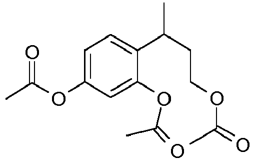 | 2 | | 4-[4-(acétyloxy)butan-2-yl]benzène-1,3-diyl diacétate |
| 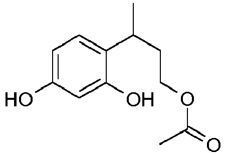 | 3 | | 3-(2,4-dihydroxyphényl)butyl acétate |

ainsi que leurs sels, leurs solvates et leurs isomères optiques, leurs racémiques, seuls ou en mélange.

Les composés de formule (I) sont nouveaux et constituent un autre objet de l'invention, ainsi que les compositions, en particulier les compositions cosmétiques, qui les contiennent ;

Les composés de l'invention de formule (I) peuvent être préparés selon le schéma 1 suivant.

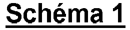

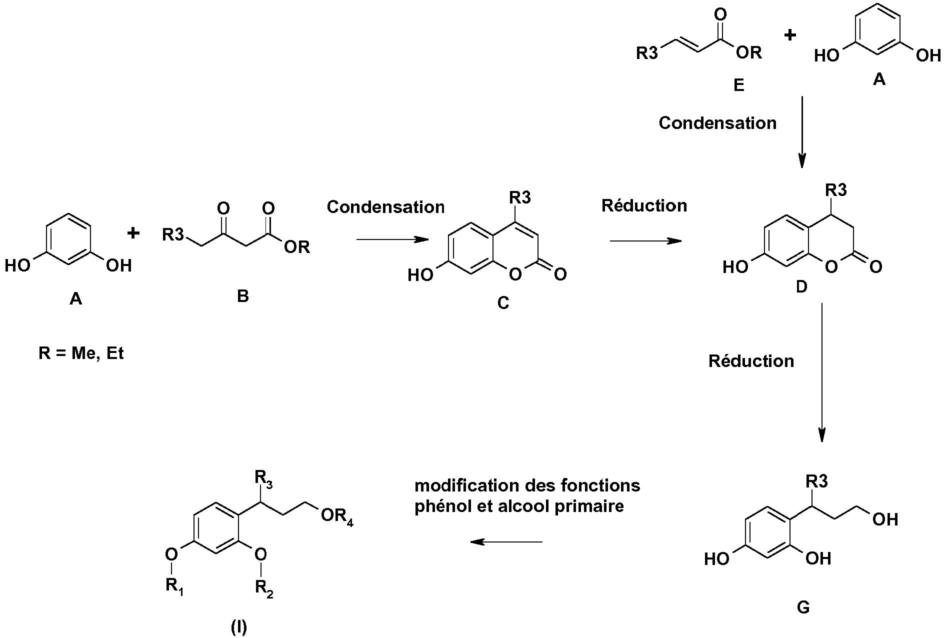

Selon ce schéma 1, la synthèse des composés (I) passe par l'intermédiaire clef de type dihydrocoumarine D dont la synthèse est entre autre décrite dans WO2005/085169. L'homme de l'art peut en adapter la stratégie en fonction des groupements R3.

La résorcine A peut réagir en présence d'un beta céto-ester B pour conduire à la coumarine C. Cette dernière est réduite par une hydrogénation catalytique selon les conditions connues de l'homme du métier pour conduire à D.

D peut également être obtenu à partir de la résorcine A en présence d'un ester alpha beta insaturé E. La fonction lactonique de D est ensuite réduite par des hydrures, après d'éventuelles modifications préalables des fonctions phénoliques par des réactions connues de l'homme du métier telles que des réactions de protection/déprotection.

L'obtention des dérivés C et D via la réaction entre (A et B) ou (A et E) peut être réalisée notamment en présence d'un solvant organique pouvant être choisi parmi le toluène, le tétrahydrofurane, l'heptane, l'isooctane, le méthyltétrahydrofurane, la méthyléthyle cétone, la méthylisobutyle cétone, le dioxane, l'acétate d'éthyle, l'acétate d'isopropyle, l'isododécane et leurs mélanges, notamment à une température comprise entre 15 et 200 °C, éventuellement en présence d'un catalyseur (acide ou basique) tel que décrit dans les publications : Synthesis of 7-hydroxycoumarins by Pechmann reaction using Nafion resin/silica nanocomposites as catalysts : Laufer MC, Hausmann H, Hölderich WF, J of catalysis, 2003, 218, 315-320 ; Synthesis of 7-hydroxycoumarins catalysed by solid acid catalysts Hoefnagel A, Gunnewegh E, Downing R, van Bekkum H, J Chem Soc Chem Commun, 1995, 225-226 ; les dérivés C et D peuvent ainsi être obtenus en particulier en présence d'un catalyseur acide tel que l'acide sulfurique, l'acide méthanesulfonique, l'acide triflique, l'acide paratoluène sulfonique, des résines sulfoniques telles que les Dowex® ou les Amberlyst® (vendues par la société Aldrich).

Les composés de formule (I) pour lesquels R₁ et ou R₂ désignent un groupe COR₅ peuvent être obtenus par acétylation/estérification. La réaction d'acétylation/estérification peut être effectuée avec l'anhydride acétique lorsque R₅ désigne un radical méthyle (ou plus généralement un anhydride R₅COOCOR₅) ou le chlorure d'acétyle lorsque R₅ désigne un radical méthyle (ou plus généralement un chlorure d'acide R5COCl), notamment en présence de solvant aprotique tel que le toluène, la pyridine, le tétrahydrofurane. La réaction d'acétylation/estérification peut être sélective en employant des groupements protecteurs sur les fonctions ne devant pas être acétylées/estérifiées et en effectuant après acétylation/esterification une réaction de déprotection, selon les techniques connues de la synthèse organique.

La réaction de réduction de la lactone D par des hydrures pour conduire au dérivé G peut être réalisée éventuellement en présence d'un solvant organique aprotique notamment le tétrahydrofurane, le dioxane, le diméthylformamide, le diméthylsulfoxyde, le 2-méthyltétrahydrofurane, le dichlorométhane , le toluène, à une température comprise entre 0 °C et 200 °C, notamment entre 20 °C et 60 °C.

L'ensemble de ces étapes peuvent également faire appel à des stratégies de protection/déprotection usuellement utilisées en chimie organique et compilées dans l'ouvrage « Protecting Groups in Organic Synthesis » Greene, Wuts, Wiley Interscience, en fonction de la nature des radicaux, afin de placer sélectivement les groupements R1, R2 et/ou R4 tels que précédemment définis à partir du composé G.

A titre illustratif du schéma de synthèse général ci-dessus, on peut par exemple synthétiser les composés de formule (I) pour lesquels R1=R2=R4 = H et R3= méthyle, respectivement R3 = Ethyle) selon les schémas 2, ou 4 (respectivement selon le schéma 3) ci-dessous :
Dans ces schémas « t.a. » signifie température ambiante.

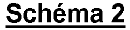

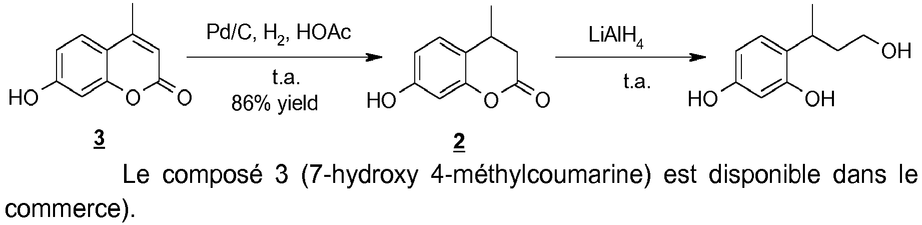

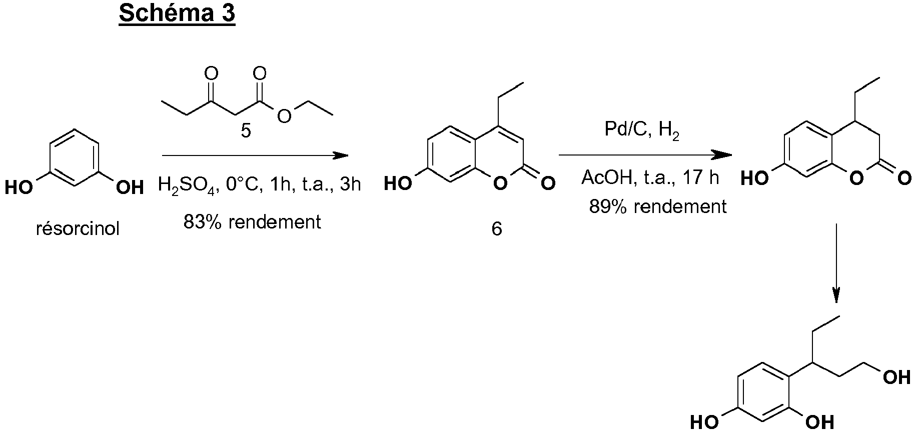

Selon le schéma 3, les réactifs, propionylacétate d'éthyle 5 et résorcinol réagissent comme décrit dans le schéma 1 ci-dessus pour donner le composé 6, qui peut être réduit par des hydrures pour conduire au composé de formule (I) correspondant. Une description de cette chimie peut être trouvée dans le document suivant Asian Journal of Chemistry, 2010, 22 (7), 5694-5698

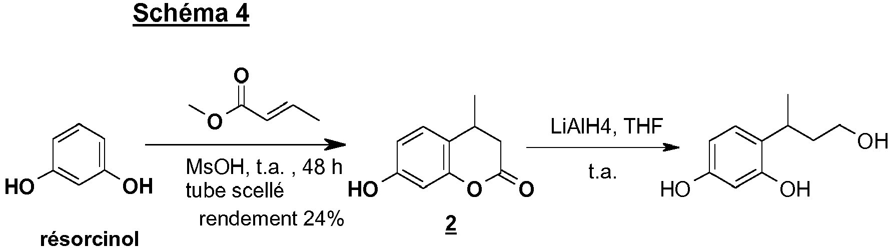

Selon le schéma 4, on met à réagir du résorcinol avec de l'ester crotonique de méthyle en présence de MSOH en excès pour donner la 7-hydroxy-4-méthyl-3,4-dihydrocoumarine 2 qui après réduction par des hydrures, conduit au composé de formule (I) correspondant.

Les composés de formule (I) selon l'invention trouvent une application toute particulière dans le domaine cosmétique.

La composition selon l'invention comprend, dans un milieu physiologiquement acceptable, un composé de formule (I) tel que décrite précédemment.

Par milieu physiologiquement acceptable, on comprend un milieu compatible avec les matières kératiniques d'êtres humains comme la peau du corps ou du visage, les lèvres, les muqueuses, les cils, les ongles, le cuir chevelu et/ou les cheveux.

Le composé (I) peut être présent dans la composition selon l'invention en une quantité qui peut être comprise entre 0,01 et 10 % en poids, de préférence entre 0,1 à 5 % en poids, notamment de 0,5 à 3 % en poids, par rapport au poids total de la composition.

La composition selon l'invention est avantageusement une composition cosmétique : elle peut comprendre des adjuvants usuellement employés dans le domaine cosmétique.

On peut notamment citer l'eau; les solvants organiques, notamment les alcools en C2-C6 ; les huiles, notamment les huiles hydrocarbonées, les huiles siliconées ; les cires, les pigments, les charges, les colorants, les tensioactifs, les émulsionnants ; les actifs cosmétiques, les filtres UV, les polymères, les épaississants, les conservateurs, les parfums, les bactéricides, les céramides, les absorbeurs d'odeur, les antioxydants.

Ces éventuels adjuvants cosmétiques peuvent être présents dans la composition à raison de 0,001 à 80 % en poids, notamment 0,1 à 40 % en poids, par rapport au poids total de la composition. En tout état de cause, ces adjuvants, ainsi que leurs proportions, seront choisis par l'homme du métier de manière telle que les propriétés avantageuses des composés selon l'invention ne soient pas, ou substantiellement pas, altérées par l'adjonction envisagée.

Comme actifs, il sera avantageux d'introduire dans la composition selon l'invention au moins un composé choisi parmi: les agents desquamants; les agents apaisants, les agents photoprotecteurs organique ou inorganique, les agents hydratants; les agents dépigmentants ; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants et/ou les agents dermo-décontractants; les agents tenseurs; les agents anti-pollution et/ou anti-radicalaire; les agents agissant sur la microcirculation; les agents agissant sur le métabolisme énergétique des cellules; et leurs mélanges.

La composition selon l'invention peut se présenter sous toutes les formes galéniques normalement utilisées dans le domaine cosmétique, et notamment sous forme d'une solution aqueuse ou hydroalcoolique, éventuellement gélifiée, d'une dispersion du type lotion éventuellement biphasée, d'une émulsion huile-dans-eau ou eau-dans-huile ou multiple ((E/H/E ou H/E/H par exemple), d'un gel aqueux, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non ionique; de gel aqueux ou huileux. Ces compositions sont préparées selon les méthodes usuelles. On préfère utiliser selon cette invention une composition sous la forme d'une émulsion notamment huile-dans-eau.

La composition selon l'invention peut constituer une composition de soin de la peau, et notamment une crème de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires); un fond de teint fluide, un lait de démaquillage, un lait corporel de protection ou de soin, un lait anti-solaire; une lotion, gel ou mousse pour le soin de la peau, comme une lotion de nettoyage.]

L'invention est illustrée plus en détail par les exemples non limitatifs suivants.

### Exemple 1 : synthèse du composé 1 :

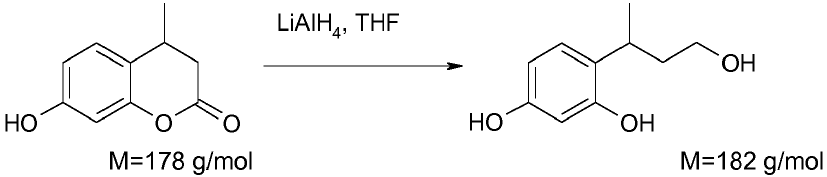

### Réactifs :

- 7-HYDROXY-4-METHYL-3,4-DIHYDRO-2H-1-BENZOPYRAN-2-ONE : 5.7 g
- LiAIH4 poudre (3 eq) : 3.7 g
- THF anhydre : 250 ml

### Mode opératoire :

Dans un ballon, on a introduit le LiAlH4, le THF (en partie), puis goutte à goutte la coumarine en solution. On a laissé sous agitation une nuit à Température ambiante.

On a refroidit le milieu réactionnel à 0°C et on a ajouté avec précaution 20 ml d'eau puis 250 ml d'HCl 1 N. Le THF a été évaporé , puis le résidu a été extrait 3 fois à l'éther diéthylique. Les phases organiques réunies ont été lavées avec une solution de NaCl saturé, puis séchées avec Na2SO4, filtrées et évaporées.

On a récupéré 5 g d'une poudre légèrement rosée correspondant au composé attendu (rendement 86 %).

Les spectres RMN 1H et de masse sont conformes à la structure.
Point de fusion : 128.8-129.4°C (tube capillaire)

### Exemple 2 : synthèse du composé 2 :

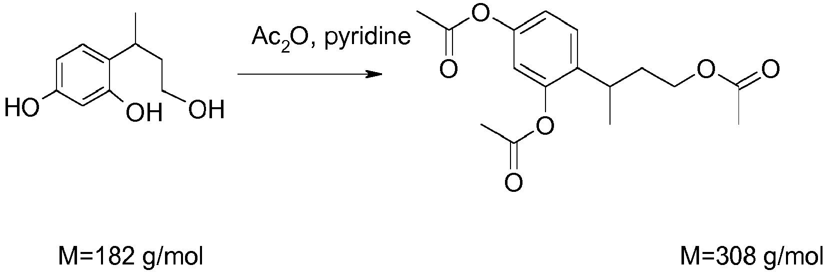

### Réactifs :

- 4-(4-hydroxybutan-2-yl)benzene-1,3-diol : 0.8 g
- Anhydride acétique (3 eq) : 1.2 ml
- Pyridine : 5 ml

### Mode opératoire :

Dans un ballon, on a introduit le 4-(4-hydroxybutan-2-yl)benzène-1,3-diol, puis la pyridine. On a refroidit le milieu à 0°C et puis on a ajouté l'anhydride acétique. On a laissé sous agitation une nuit à température ambiante.

On a ajouté 50 ml d'acétate d'éthyle et 50 ml HCl 1 N. On a lavé la phase organique avec 2 fois 50 ml HCl 1 N , puis 50 ml d'eau et 50 ml d'une solution saturée de NaCl. La phase organique a été séchée avec Na2SO4, filtrée et évaporée.

On a récupéré 1 g d'une huile jaune correspondant au composé attendu. (rendement 77 %).

Les spectres RMN 1H et de masse sont conformes à la structure.

### Exemple 3 : synthèse du composé 3 :

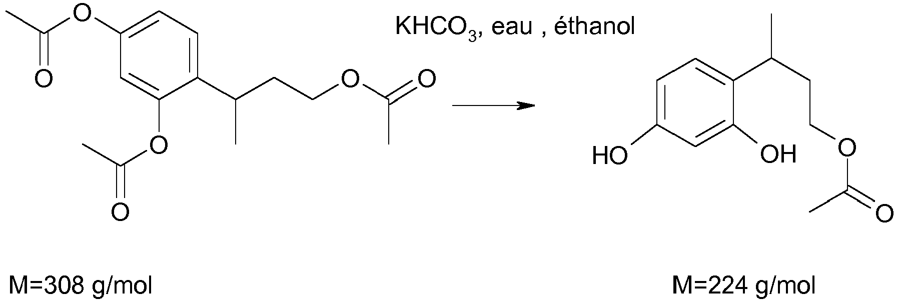

### Réactifs :

- 4-[4-(acétyloxy)butan-2-yl]benzène-1,3-diyl diacétate : 0.4 g
- Hydrogénocarbonate de potassium (2 eq) : 260 mg
- Eau : 1 ml
- Ethanol : 2 ml

### Mode opératoire :

Dans un ballon, on a introduit le 4-[4-(acétyloxy)butan-2-yl]benzene-1,3-diyl diacétate et l'éthanol, puis on a ajouté l'eau et le KHCO3.

On a laissé 1h à température ambiante. On a évaporé l'éthanol et on a extrait à l'acétate d'éthyle. La phase organique a été séchée avec Na2SO4, puis filtrée et évaporée.

On a récupéré 0, 25 g d'une huile jaune correspondant au composé attendu . (rendement 86 %).

Les spectres RMN 1H et de masse sont conformes à la structure attendue.

### Exemple 4 : Mise en évidence de l'activité sur la mélanogénèse constitutive

L'efficacité a été démontrée sur la base du test suivant :
Les évaluations de l'effet de prévention ou de diminution de la pigmentation de la peau et / ou de l'éclaircissement de celle-ci peau les exemples sont réalisés de la manière suivante.

La mesure de l'activité dépigmentante (réduction de la production de mélanine) de composés de formule (I) a été effectuée par dosage des mélanocytes humains normaux in vitro comme suit.

Tout d'abord, des mélanocytes humains normaux sont cultivés et distribués dans 384puits. Après 24 heures, le milieu de culture a été remplacé par un milieu contenant des composés de formule (I) à évaluer. Les cellules ont été incubées 72 heures avant la mesure de la densité optique finale qui mesure la quantité de mélanine produite par les mélanocytes. Un effet dose est mis en œuvre en utilisant une large gamme de concentration des composés évalués. Ainsi, en faisant correspondre les concentrations et les mesures de mélanine, il est possible de déterminer une CI50 en µM: concentration à laquelle 50 % de diminution de la synthèse de mélanine est atteinte.

Les composés de formule (I) ont montré un effet dépigmentant fort.

| N° Composé | IC50 (µM) | Concentration maximale testée (µM) |
|---|---|---|
| 1 | 3,61 | 200 |
| 2 | 4,08 | 200 |
| 3 | 4,96 | 200 |

Ces résultats ont été comparés à ceux obtenus avec le composé le plus proche décrit dans l'art antérieur, dans le brevet JP 2007186445 de Kuraray

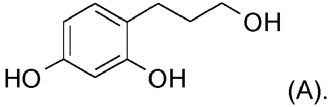

Pour ce composé (A), la valeur de l'IC50 est de 23,4 µM concentration maximale testée 200µM.

Les composés de l'invention ont une activité sur la réduction de la mélanogénèse beaucoup plus importante que celle du composé (A) hors invention ;

### Exemple 5 : Composition cosmétique

On prépare une composition dépigmentante pour la peau comprenant (en gramme) :

| | |
|---|---|
| Composé n°1 | 2 g |
| PEG400 | 68 g |
| Ethanol | 30 g |

La composition appliquée sur la peau permet d'estomper les taches brunes.

### Exemple 6 : gel

On prépare un gel dépigmentant pour la peau comprenant (% en poids) :

| | |
|---|---|
| Composé n°2 | 0,25% |
| Carbomer (Carbopol 981 de chez Lubrizol) | 1% |
| conservateur | qs |
| eau | qsp 100 % |

La composition appliquée sur la peau permet d'estomper les taches brunes.

### Exemple 7 : Mise en évidence de l'activité dépigmentante sur un épiderme reconstruit pigmenté

Le but de ce test est d'évaluer la modulation de la mélanogénèse dans les épidermes reconstruits pigmentés après application « systémique » des produits.
- Les composés sont testés à 30µM dans le DMSO.
- Les épidermes pigmentés sont reconstruits à l'aide de kératinocytes et mélanocytes d'origine européenne ensemencés sur le substrat BPER (EPISKIN). Les produits à tester sont ajoutés au milieu de culture dès l'ensemencement des cellules et à tous les changements de milieu.

Le modèle d'étude type épiderme reconstruit pigmenté a été publié par :
Regnier M, Duval C, Galey JB, Philippe M, Lagrange A, Tuloup R, Schmidt R, Cellular and Molecular Biology, 1999, 45, 7, 969-980 : « Keratinocyte-Melanocyte co-cultures and pigmented reconstructed human epidermis : models to study modulation of melanogenesis ».

La mélanine a été quantifiée par analyse d'images sur coupes histologiques après révélation par coloration Fontana Masson. Chaque épiderme coloré est photographié sur toute sa longueur à l'aide d'une caméra reliée à un microscope.

Les résultats sont rassemblés dans le tableau suivant :
La diminution de la quantité de mélanine est évaluée par rapport au solvant (DMSO)

| | **Activité dépigmentante/DMSO** |
|---|---|
| Lucinol | -41% |
| Composé 1 | -59% |

Les résultats obtenus montrent que le composé 1 selon l'invention a une action dépigmentante plus importante que celle du lucinol.

## Revendications

1. Utilisation cosmétique non thérapeutique d'au moins un composé de formule (I) comme agent blanchissant, éclaircissant et/ou dépigmentant des matières kératiniques, notamment de la peau 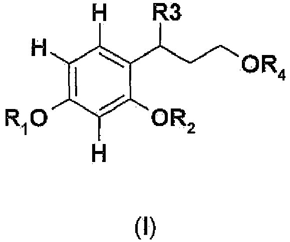 dans laquelle:
R₁, R₂ : identiques ou différents, désignent
a) hydrogène,
b) un radical COR₅ dans lequel R₅ désigne un radical alkyle linéaire en C₁-C₁₀ ou ramifié en C₃-C₁₀, de préférence un radical alkyle linéaire en C₁-C₆ ou ramifié en C₃-C₆, plus préférentiellement un radical alkyle linéaire en C₁-C₄ R₃ désigne un radical alkyl linéaire en C₁-C₆ ou ramifié en C₃-C₆, de préférence un radical alkyle linéaire en C₁-C₄ ou ramifié en C₃-C₄
R₄ désigne
a) H,
b) un radical alkyle linéaire en C₁-C₆ ou ramifié en C₃-C₆,
c) un radical COR₅,
ainsi que leurs sels, leurs solvates et leurs isomères optiques, leurs racémiques, seuls ou en mélange.

2. Utilisation selon la revendication 1, **caractérisée en ce que** R4 désigne H ou un radical alkyl linéaire en C₁-C₄, un radical alkyl ramifié en C₃-C₄ ou un radical COR₅, plus préférentiellement H ou un radical COR₅.

3. Utilisation selon l'une des revendications 1 ou 2, **caractérisée en ce que** les composés de formule (I) sont choisis parmi ceux pour lesquels :
R₁, R₂, identiques ou différents, désignent H ou un radical COCH₃, et de préférence R₁ et R₂ sont identiques.
R₃ désigne méthyle, éthyle ou isopropyle, et
R₄ désigne H ou un radical COCH₃
ainsi que leurs sels, leurs solvates et leurs isomères optiques, leurs racémiques, seuls ou en mélange.

4. Utilisation selon l'une des revendications 1 à 3, **caractérisée en ce que** les composés de formule (I) sont choisis parmi ceux pour lesquels :
R₁, R₂, identiques ou différents, désignent H ou un radical COCH₃, et de préférence R₁ et R₂ sont identiques,
R₃ désigne méthyle, et
R₄ désigne H ou un radical COCH₃,
ainsi que leurs sels, leurs solvates et leurs isomères optiques, leurs racémiques.

5. Utilisation selon l'une des revendications 1 à 4, **caractérisée en ce que** les composés de formule (I) sont choisis parmi :
| Structure | Composé n° | Nom chimique |
|---|---|---|
| 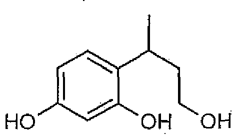 | 1 | 4-(3-Hydroxy-1-méthyl-propyl)-benzène-1,3-diol |
| 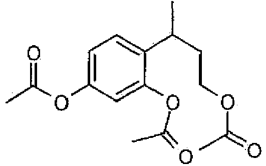 | 2 | 4-[4-(acétyloxy)butan-2-yl]benzène-1,3-diyl diacétate |
| 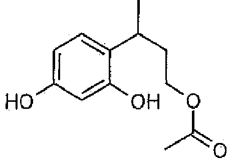 | 3 | 3-(2,4-dihydroxyphényl)butyl acétate |
ainsi que leurs sels, leurs solvates et leurs isomères optiques, leurs racémiques, seuls ou en mélange.

6. Composés de formule (I) suivante : 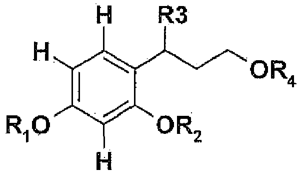 dans laquelle:
R₁, R₂ : identiques ou différents, désignent
a) hydrogène,
b) un radical COR₅ dans lequel R₅ désigne un radical alkyle linéaire en C₁-C₁₀ ou ramifié en C₃-C₁₀, de préférence un radical alkyle linéaire en C₁-C₆ ou ramifié en C₃-C₆, plus préférentiellement un radical alkyle linéaire en C₁-C₄,
R₃ désigne un radical alkyl linéaire en C₁-C₆ ou ramifié en C₃-C₆, de préférence un radical alkyle linéaire en C₁-C₄ ou ramifié en C₃-C₄
R₄ désigne
a) H,
b) un radical alkyl linéaire en C₁-C₆ ou ramifié en C₃-C₆,
c) un radical COR₅ dans lequel R₅ désigne un radical alkyle linéaire en C₁-C₁₀ ou ramifié en C₃-C₁₀, de préférence un radical alkyle linéaire en C₁-C₆ ou ramifié en C₃-C₆, plus préférentiellement un radical alkyle linéaire en C₁-C₄,
ainsi que leurs sels, leurs solvates et leurs isomères optiques, leurs racémiques, seuls ou en mélange.

7. Composé de formule (I) selon la revendication 6, **caractérisé en ce que** R₄ désigne H ou un radical alkyl linéaire en C₁-C₄, un radical alkyl ramifié en C₃-C₄ ou un radical COR₅, plus préférentiellement H ou un radical COR₅.

8. Composé de formule (I) selon l'une des revendications 6 ou 7, **caractérisé en ce que**:
R₁, R₂, identiques ou différents, désignent H ou un radical COCH₃, et de préférence R₁ et R₂ sont identiques.
R₃ désigne méthyle, éthyle ou isopropyle, et
R₄ désigne H ou un radical COCH₃
ainsi que leurs sels, leurs solvates et leurs isomères optiques, leurs racémiques, seuls ou en mélange.

9. Composé de formule (I) selon l'une des revendications 6 à 8, **caractérisé en ce que**:
R₁, R₂, identiques ou différents, désignent H ou un radical COCH₃, et de préférence R₁ et R₂ sont identiques,
R₃ désigne méthyle, et
R₄ désigne H ou un radical COCH₃,
ainsi que leurs sels, leurs solvates et leurs isomères optiques, leurs racémiques.

10. Composé de formule (I) selon l'une des revendications 6 à 9, **caractérisé en ce que** les composés de formule (I) sont choisis parmi :
| Structure | Composé n° | Nom chimique |
|---|---|---|
| 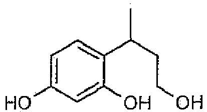 | 1 | 4-(3-Hydroxy-1-méthyl-propyl)-benzene-1,3-diol |
| 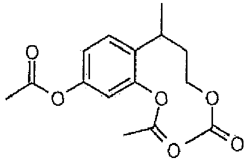 | 2 | 4-[4-(acétyloxy)butan-2-yl]benzène-1,3-diyl diacétate |
| 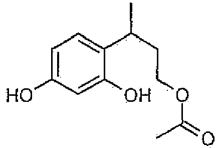 | 3 | 3-(2,4-dihydroxyphényl)butyl acétate |
ainsi que leurs sels, leurs solvates et leurs isomères optiques, leurs racémiques, seuls ou en mélange.

11. Composition comprenant, dans un milieu physiologiquement acceptable, au moins un composé de formule (I) selon l'une quelconque des revendications 6 à 10.

12. Composition selon la revendication précédente, **caractérisée en ce que** ledit composé de formule (I) est présent en une quantité comprise entre 0,01 et 10 % en poids, de préférence entre 0,1 à 5 % en poids, notamment de 0,5 à 3 % en poids, par rapport au poids total de la composition.

13. Procédé cosmétique non thérapeutique de dépigmentation, d'éclaircissement et/ou de blanchiment des matières kératiniques, notamment de la peau, comprenant l'application de la composition selon la revendication 11 ou 12.

## Patentansprüche

1. Nichttherapeutische kosmetische Verwendung von mindestens einer Verbindung der Formel (I) als Mittel zur Bleichung, Aufhellung und/oder Depigmentierung von Keratinmaterialien, insbesondere der Haut, 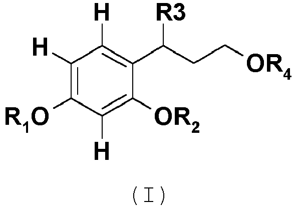 in der:
R₁ und R₂ gleich oder verschieden sind und für
a) Wasserstoff,
b) einen COR₅-Rest, in dem R₅ für einen linearen C₁-C₁₀- oder verzweigten C₃-C₁₀-Alkylrest, vorzugsweise einen linearen C₁-C₆- oder verzweigten C₃-C₆-Alkylrest, weiter bevorzugt einen linearen C₁-C₄-Alkylrest,
stehen,
R₃ für einen linearen C₁-C₆- oder verzweigten C₃-C₆-Alkylrest, vorzugsweise einen linearen C₁-C₄- oder verzweigten C₃-C₄-Alkylrest, steht,
R₄ für
a) H,
b) einen linearen C₁-C₆- oder verzweigten C₃-C₆-Alkylrest,
c) einen COR₅-Rest
steht,
sowie Salzen davon, Solvaten davon und optischen Isomeren davon und Racematen davon, alleine oder als Mischung.

2. Verwendung nach Anspruch 1, **dadurch gekennzeichnet, dass** R₄ für H oder einen linearen C₁-C₄-Alkylrest, einen verzweigten C₃-C₄-Alkylrest oder einen COR₅-Rest, weiter bevorzugt H oder einen COR₅-Rest, steht.

3. Verwendung nach Anspruch 1 oder 2, **dadurch gekennzeichnet, dass** die Verbindungen der Formel (I) aus denjenigen ausgewählt sind, für die:
R₁ und R₂ gleich oder verschieden sind und für H oder einen COCH₃-Rest stehen und vorzugsweise R₁ und R₂ gleich sind,
R₃ für Methyl, Ethyl oder Isopropyl steht und
R₄ für H oder einen COCH₃-Rest steht,
sowie Salzen davon, Solvaten davon und optischen Isomeren davon und Racematen davon, alleine oder als Mischung.

4. Verwendung nach einem der Ansprüche 1 bis 3, **dadurch gekennzeichnet, dass** die Verbindungen der Formel (I) aus denjenigen ausgewählt sind, für die:
R₁ und R₂ gleich oder verschieden sind und für H oder einen COCH₃-Rest stehen und vorzugsweise R₁ und R₂ gleich sind,
R₃ für Methyl steht und
R₄ für H oder einen COCH₃-Rest steht,
sowie Salzen davon, Solvaten davon und optischen Isomeren davon und Racematen davon, alleine oder als Mischung.

5. Verwendung nach einem der Ansprüche 1 bis 4, **dadurch gekennzeichnet, dass** die Verbindungen der Formel (I) aus
| Struktur | Verbindung Nr. | Chemischer Name |
|---|---|---|
| 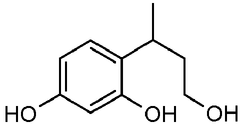 | 1 | 4-(3-Hydroxy-1-methylpropyl)benzol-1,3-diol |
| 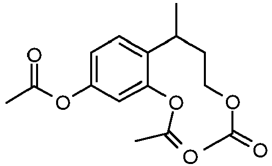 | 2 | 4-[4-(Acetyloxy)but-2-yl]benzyl-1,3-diyldiacetat |
| 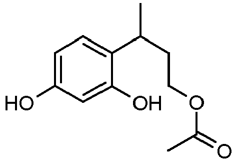 | 3 | 3-(2,4-Dihydroxyphenyl)butylacetat |
sowie Salzen davon, Solvaten davon und optischen Isomeren davon und Racematen davon, alleine oder als Mischung
ausgewählt sind.

6. Verbindung der folgenden Formel (I): 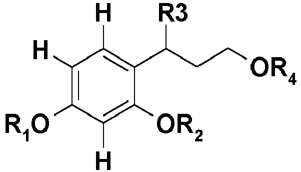 in der:
R₁ und R₂ gleich oder verschieden sind und für
a) Wasserstoff,
b) einen COR₅-Rest, in dem R₅ für einen linearen C₁-C₁₀- oder verzweigten C₃-C₁₀-Alkylrest, vorzugsweise einen linearen C₁-C₆- oder verzweigten C₃-C₆-Alkylrest, weiter bevorzugt einen linearen C₁-C₄-Alkylrest,
stehen,
R₃ für einen linearen C₁-C₆- oder verzweigten C₃-C₆-Alkylrest, vorzugsweise einen linearen C₁-C₄- oder verzweigten C₃-C₄-Alkylrest, steht,
R₄ für
a) H,
b) einen linearen C₁-C₆- oder verzweigten C₃-C₆-Alkylrest,
c) einen COR₅-Rest, in dem R₅ für einen linearen C₁-C₁₀- oder verzweigten C₃-C₁₀-Alkylrest, vorzugsweise einen linearen C₁-C₆- oder verzweigten C₃-C₆-Alkylrest, weiter bevorzugt einen linearen C₁-C₄-Alkylrest, steht,
steht,
sowie Salze davon, Solvate davon und optische Isomere davon und Racemate davon, alleine oder als Mischung.

7. Verbindung der Formel (I) nach Anspruch 6, **dadurch gekennzeichnet, dass** R₄ für H oder einen linearen C₁-C₄-Alkylrest, einen verzweigten C₃-C₄-Alkylrest oder einen COR₅-Rest, weiter bevorzugt H oder einen COR₅-Rest, steht.

8. Verbindung der Formel (I) nach Anspruch 6 oder 7, **dadurch gekennzeichnet, dass**:
R₁ und R₂ gleich oder verschieden sind und für H oder einen COCH₃-Rest stehen und vorzugsweise R₁ und R₂ gleich sind,
R₃ für Methyl, Ethyl oder Isopropyl steht und
R₄ für H oder einen COCH₃-Rest steht,
sowie Salze davon, Solvate davon und optische Isomere davon und Racemate davon, alleine oder als Mischung.

9. Verbindung der Formel (I) nach einem der Ansprüche 6 bis 8, **dadurch gekennzeichnet, dass**:
R₁ und R₂ gleich oder verschieden sind und für H oder einen COCH₃-Rest stehen und vorzugsweise R₁ und R₂ gleich sind,
R₃ für Methyl steht und
R₄ für H oder einen COCH₃-Rest steht,
sowie Salze davon, Solvate davon und optische Isomere davon und Racemate davon, alleine oder als Mischung.

10. Verbindung der Formel (I) nach einem der Ansprüche 6 bis 9, **dadurch gekennzeichnet, dass** die Verbindungen der Formel (I) aus
| Struktur | Verbindung Nr. | Chemischer Name |
|---|---|---|
| 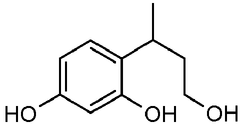 | 1 | 4-(3-Hydroxy-1-methylpropyl)benzol-1,3-diol |
| 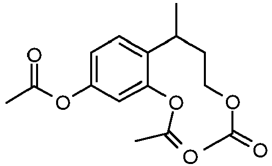 | 2 | 4-[4-(Acetyloxy)but-2-yl]benzyl-1,3-diyldiacetat |
| 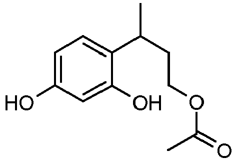 | 3 | 3-(2,4-Dihydroxyphenyl)butylacetat |
sowie Salzen davon, Solvaten davon und optischen Isomeren davon und Racematen davon, alleine oder als Mischung
ausgewählt sind.

11. Zusammensetzung, die in einem physiologisch unbedenklichen Medium mindestens eine Verbindung der Formel (I) nach einem der Ansprüche 6 bis 10 umfasst.

12. Zusammensetzung nach dem vorhergehenden Anspruch, **dadurch gekennzeichnet, dass** die Verbindung der Formel (I) in einer Menge zwischen 0,01 und 10 Gew.-%, vorzugsweise zwischen 0,1 und 5 Gew.-%, insbesondere von 0,5 bis 3 Gew.-%, bezogen auf das Gesamtgewicht der Zusammensetzung, vorliegt.

13. Nichttherapeutisches kosmetisches Verfahren zur Depigmentierung, Aufhellung und/oder Bleichung von Keratinmaterialien, insbesondere der Haut, umfassend das Aufbringen der Zusammensetzung nach Anspruch 11 oder 12.

## Claims

1. Non-therapeutic cosmetic use of at least one compound of formula (I) as an agent for bleaching, lightening and/or depigmenting keratin materials, especially the skin: 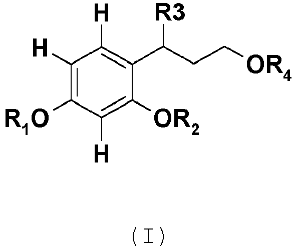 in which:
R₁ and R₂, which may be identical or different, denote:
a) hydrogen,
b) a COR₅ radical in which R₅ denotes a linear C₁-C₁₀ or branched C₃-C₁₀ alkyl radical, preferably a linear C₁-C₆ or branched C₃-C₆ alkyl radical, more preferentially a linear C₁-C₄ alkyl radical,
R₃ denotes a linear C₁-C₆ or branched C₃-C₆ alkyl radical, preferably a linear C₁-C₄ or branched C₃-C₄ alkyl radical,
R₄ denotes
a) H,
b) a linear C₁-C₆ or branched C₃-C₆ alkyl radical,
c) a COR₅ radical,
and also the salts thereof, the solvates thereof and the optical isomers thereof, and the racemates thereof, alone or as a mixture.

2. Use according to Claim 1, **characterized in that** R₄ denotes H or a linear C₁-C₄ alkyl radical, a branched C₃-C₄ alkyl radical or a COR₅ radical, more preferentially H or a COR₅ radical.

3. Use according to either of Claims 1 and 2, **characterized in that** the compounds of formula (I) are chosen from those for which:
R₁ and R₂, which may be identical or different, denote H or a COCH₃ radical, and preferably R₁ and R₂ are identical,
R₃ denotes methyl, ethyl or isopropyl, and
R₄ denotes H or a COCH₃ radical,
and also the salts thereof, the solvates thereof and the optical isomers thereof, and the racemates thereof, alone or as a mixture.

4. Use according to one of Claims 1 to 3, **characterized in that** the compounds of formula (I) are chosen from those for which:
R₁ and R₂, which may be identical or different, denote H or a COCH₃ radical, and preferably R₁ and R₂ are identical,
R₃ denotes methyl, and
R₄ denotes H or a COCH₃ radical,
and also the salts thereof, the solvates thereof and the optical isomers thereof, and the racemates thereof.

5. Use according to one of Claims 1 to 4, **characterized in that** the compounds of formula (I) are chosen from:
| Structure | Compound No. | Chemical name |
|---|---|---|
| 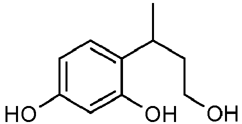 | 1 | 4-(3-hydroxy-1-methylpropyl) benzene-1,3-diol |
| 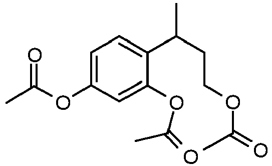 | 2 | 4-[4-(acetyloxy)but-2-yl]benzene-1,3-diyl diacetate |
| 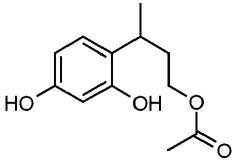 | 3 | 3-(2,4-dihydroxyphenyl) butyl acetate |
and also the salts thereof, the solvates thereof and the optical isomers thereof, and the racemates thereof, alone or as a mixture.

6. Compounds of formula (I) below: 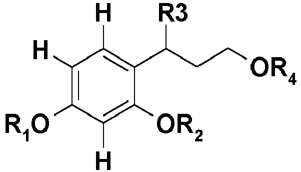 in which:
R₁ and R₂, which may be identical or different, denote:
a) hydrogen,
b) a COR₅ radical in which R₅ denotes a linear C₁-C₁₀ or branched C₃-C₁₀ alkyl radical, preferably a linear C₁-C₆ or branched C₃-C₆ alkyl radical, more preferentially a linear C₁-C₄ alkyl radical,
R₃ denotes a linear C₁-C₆ or branched C₃-C₆ alkyl radical, preferably a linear C₁-C₄ or branched C₃-C₄ alkyl radical,
R₄ denotes
a) H,
b) a linear C₁-C₆ or branched C₃-C₆ alkyl radical,
c) a COR₅ radical in which R₅ denotes a linear C₁-C₁₀ or branched C₃-C₁₀ alkyl radical, preferably a linear C₁-C₆ or branched C₃-C₆ alkyl radical, more preferentially a linear C₁-C₄ alkyl radical,
and also the salts thereof, the solvates thereof and the optical isomers thereof, and the racemates thereof, alone or as a mixture.

7. Compound of formula (I) according to Claim 6, **characterized in that** R₄ denotes H or a linear C₁-C₄ alkyl radical, a branched C₃-C₄ alkyl radical or a COR₅ radical, more preferentially H or a COR₅ radical.

8. Compound of formula (I) according to either of Claims 6 and 7, **characterized in that**:
R₁ and R₂, which may be identical or different, denote H or a COCH₃ radical, and preferably R₁ and R₂ are identical,
R₃ denotes methyl, ethyl or isopropyl, and
R₄ denotes H or a COCH₃ radical,
and also the salts thereof, the solvates thereof and the optical isomers thereof, and the racemates thereof, alone or as a mixture.

9. Compound of formula (I) according to one of claims 6 to 8, **characterized in that**:
R₁ and R₂, which may be identical or different, denote H or a COCH₃ radical, and preferably R₁ and R₂ are identical,
R₃ denotes methyl, and
R₄ denotes H or a COCH₃ radical,
and also the salts thereof, the solvates thereof and the optical isomers thereof, and the racemates thereof.

10. Compound of formula (I) according to one of claims 6 to 9, **characterized in that** the compounds of formula (I) are chosen from:
| Structure | Compound No. | Chemical name |
|---|---|---|
| 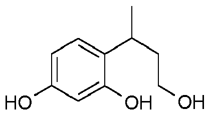 | 1 | 4-(3-hydroxy-1-methylpropyl)benzene-1,3-diol |
| 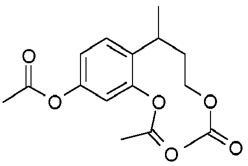 | 2 | 4-[4-(acetyloxy)but-2-yl]benzene-1,3-diyl diacetate |
| 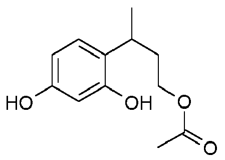 | 3 | 3-(2,4-dihydroxyphenyl) butyl acetate |
and also the salts thereof, the solvates thereof and the optical isomers thereof, and the racemates thereof, alone or as a mixture.

11. Composition comprising, in a physiologically acceptable medium, at least one compound of formula (I) according to any one of Claims 6 to 10.

12. Composition according to the preceding claim, **characterized in that** said compound of formula (I) is present in an amount of between 0.01% and 10% by weight, preferably between 0.1% and 5% by weight and in particular from 0.5% to 3% by weight, relative to the total weight of the composition.

13. Non-therapeutic cosmetic process for depigmenting, lightening and/or bleaching keratin materials, especially the skin, comprising the application of the composition according to Claim 11 or 12.
